# EUROPEAN PATENT APPLICATION

(11) **EP 4 401 086 A1**
(43) Date of publication of application: **17.07.2024**
(21) Application number: 23156457.6
(22) Date of filing: 14.02.2023
(51) Int. Cl.: G16H 40/63, A61B 5/00

(54) **METHODS AND SYSTEMS FOR COMPACT DISPLAY OF PHYSIOLOGICAL PARAMETERS**

(30) Priority: 13.01.2023 US 202363438911 P
(71) Applicant: Koninklijke Philips N.V., 5656 AG Eindhoven (NL)
(72) Inventor: KACZMAREK, Piotr, Eindhoven (NL); DANISMAN-TASAR, Mine, Eindhoven (NL); LOPEZ, Benjamin, 5656AG Eindhoven (NL); HUIJBREGTS, Laurentia Johanna, Eindhoven (NL); VAN LIESHOUT, Ron Martinus Laurentius, Eindhoven (NL)
(74) Representative: Philips Intellectual Property & Standards

(57) **Abstract**

A method (100) for displaying a visual representation of data, comprising: receiving (120) data representing a measured parameter for a plurality of time points; comparing (130) the received data representing a measured parameter to one or more predetermined thresholds at each of the plurality of time points, wherein the one or more predetermined thresholds comprises a threshold separating a reference range from a first alert range; converting (140), based on the comparison, the received data into a first visual data representation, wherein the first visual data representation comprises one of: (i) a reference range location on a visual graph; or (ii) a first alert range location on the visual graph, wherein the first alert range location on the visual graph is above or below the reference range location on the visual graph; and displaying (150) the first visual data representation on a display.

## Description

### FIELD OF THE DISCLOSURE

The present disclosure is directed generally to methods and systems for displaying a visual representation of patient data using an efficient and compact display method.

### BACKGROUND OF THE INVENTION

In the healthcare setting, caregivers must be able to compare trends of multiple parameters, often together with the interventions, in one glance. It is advantageous to view as many vital trends as possible at once in order to quickly gather a snapshot of the overall patient condition, which is important since vital signs can be interrelated. However, a display comprising a full graph for each vital trend requires significant space and thus only a few full graphs will fit a screen.

Existing systems provide trends of multiple parameters on one screen by showing multiple trendlines per graph, where some trendlines are connected to the left vertical scale and others to the right vertical scale, and allow, for example, two of such graphs on a page. However, this results in several problems including crossing lines, a messy view, and inability to see which parameter is depicted where on the graph or the screen. Further, it is not easy to see when a parameter is within the normal range and when it is outside the normal range.

For example, one solution is to show when a parameter is in a normal range, out of the normal range, and far out of the normal range, but these solutions fail to show whether the parameter is above the normal range or below the normal range when that parameter is outside the normal range. This is a vital distinction for a caregiver, because different interventions may be necessary depending on whether the parameter is above the normal range or below the normal range. For example, intervention will be different when blood pressure is too high (above the normal range) and when the blood pressure is too low (below the normal range). However, prior art systems do not provide both the information about when the parameter is inside or outside the normal range and whether the parameter is above or below that normal range.

### SUMMARY OF THE INVENTION

Accordingly, there is a continued need for methods and systems capable of quickly displaying information about data for a plurality of parameters over time, including if and when the patient data for each parameter is inside or outside the normal range for that parameter, and whether the patient data is above or below that normal range when it is outside the normal range for that parameter.

Various embodiments and implementations herein are directed to a data display system configured to display a visual representation of data for a plurality of parameters. The data display system receives data for a plurality of time points, wherein the data is variable over time. The system compares the received data to a predetermined threshold at each of the plurality of time points, the threshold comprises a threshold separating an expected reference range from a first alert range. The system converts the received data into a visual data representation. According to an embodiment, the visual data representation comprises at least one of: (i) a reference expected range location on a visual graph; or (ii) a first alert range location on the visual graph, where the first alert range location on the visual graph is above or below the reference expected range location on the visual graph. The data display system then displays the generated visual data representation on a display.

Generally, in one aspect, a method for displaying a visual representation of data is provided. The method includes: receiving data representing a measured parameter for a plurality of time points, wherein the data is variable over time; comparing the received data representing a measured parameter to one or more predetermined thresholds at each of the plurality of time points, wherein the one or more predetermined thresholds comprises a threshold separating a reference range from a first alert range; converting the received data into a first visual data representation, wherein the first visual data representation comprises one of: (i) a reference range location on a visual graph when the data is within the reference range; or (ii) a first alert range location on the visual graph when the data is within the first alert range, wherein the first alert range location on the visual graph is above or below the reference range location on the visual graph; and displaying the first visual data representation on a display. According to an embodiment, the method further includes repetitions to display a plurality of converted visual data representations on the display, each visual data representation representing a different measured parameter.

According to an embodiment, the first alert range is an alarm range.

According to an embodiment, the greater a difference of a value of the data relative to the reference range, the further away from the reference range the data is placed within the first alert range in the first visual data representation.

According to an embodiment, the first visual data representation comprises a first color, pattern, brightness, darkness, or thickness when the data is within the reference range, and comprises a second color, pattern, brightness, darkness, or thickness when the data is within the first alert range, the first color, pattern, brightness, darkness, or thickness and second color, pattern, brightness, darkness, or thickness being different colors, patterns, brightness, darkness, or thickness. According to an embodiment, the first color is gray, and the second color is yellow or red.

According to an embodiment, the one or more predetermined thresholds further comprises a threshold identifying a second alert range, wherein all of the data is within the reference range, the first alert range, or the second alert range; and wherein the first visual data representation can further comprise: (iii) a second alert range location on the visual graph when the data is within the second alert range.

According to an embodiment, the second alert range is located above or below the reference range location on the visual graph, on a side of the reference range opposite the first alert range.

According to an embodiment, the first alert range is located between the reference range and the second alert range on the visual graph.

According to an embodiment, the first alert range and/or the second alert range is an alarm range.

According to an embodiment, the first alert range and the second alert range are unequal ranges relative to each other.

According to an embodiment, the method further includes displaying, on the display, an interactive option to change the display to provide exact values for the data in the first visual data representation.

According to another aspect, a system for displaying a visual representation of data is provided. The system includes: data representing a measured parameter for a plurality of time points, wherein the data is variable over time; a processor configured to: receive the data; compare the received data to one or more predetermined thresholds at each of the plurality of time points, wherein the one or more predetermined thresholds comprises a threshold separating a reference range from a first alert range; convert the data into a first visual data representation, wherein the first visual data representation comprises one of: (i) a reference range location on a visual graph when the data is within the reference range; or (ii) a first alert range location on the visual graph when the data is within the first alert range, wherein the first alert range location on the visual graph is above or below the reference range location on the visual graph; and a user interface configured to display the visual data representation.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a method for displaying a visual representation of data, in accordance with an embodiment.
Fig. 2 is a schematic representation of a data display system, in accordance with an embodiment.
Fig. 3A is a schematic representation of a visual representation of data, in accordance with an embodiment.
Fig. 3B is a schematic representation of a visual representation of data, in accordance with an embodiment.
Fig. 4 is a schematic representation of an expanded view of patient data, in accordance with an embodiment.
Fig. 5 is a schematic representation of a collapsed view of patient data, in accordance with an embodiment.
Fig. 6 is a schematic representation comparing an expanded view of patient data and a collapsed view of patient data, in accordance with an embodiment.
Fig. 7A is a schematic representation of patient data, in accordance with an embodiment.
Fig. 7B is a schematic representation of patient data, in accordance with an embodiment.
Fig. 8A is a schematic representation of collapsed data, in accordance with an embodiment.
Fig. 8B is a schematic representation of collapsed data, in accordance with an embodiment.
Fig. 9A is a schematic representation of collapsed data, in accordance with an embodiment.
Fig. 9B is a schematic representation of collapsed data, in accordance with an embodiment.
Fig. 10 is a schematic representation of a portion of a collapsed patient data graph, in accordance with an embodiment.

### DETAILED DESCRIPTION OF EMBODIMENTS

The present disclosure describes various embodiments of a system and method configured to display a visual representation of data for a plurality of parameters. The data display system receives data for a plurality of time points, wherein the data is variable over time. The system compares the received data to a predetermined threshold at each of the plurality of time points, the threshold comprises a threshold separating an expected reference range from a first alert range. The system converts the received data into a visual data representation. According to an embodiment, the visual data representation comprises at least one of: (i) a reference expected range location on a visual graph; or (ii) a first alert range location on the visual graph, where the first alert range location on the visual graph is above or below the reference expected range location on the visual graph. The data display system then displays the generated visual data representation on a display.

The embodiments and implementations disclosed or otherwise envisioned herein can be utilized with a patient care system, including but not limited to clinical decision support tools, patient monitoring systems, and other systems. For example, one application of the embodiments and implementations herein is to improve analysis and display systems such as, e.g., the Philips^{®} patient monitoring systems and products (manufactured by Koninklijke Philips, N.V.), among many other products. However, the disclosure is not limited to these devices or systems, and thus disclosure and embodiments disclosed herein can encompass any device or system capable of monitoring patients and/or displaying patient monitoring information.

For example, according to another embodiment, the embodiments and implementations disclosed or otherwise envisioned herein can be utilized with any system that includes variable data over time and data visualization.

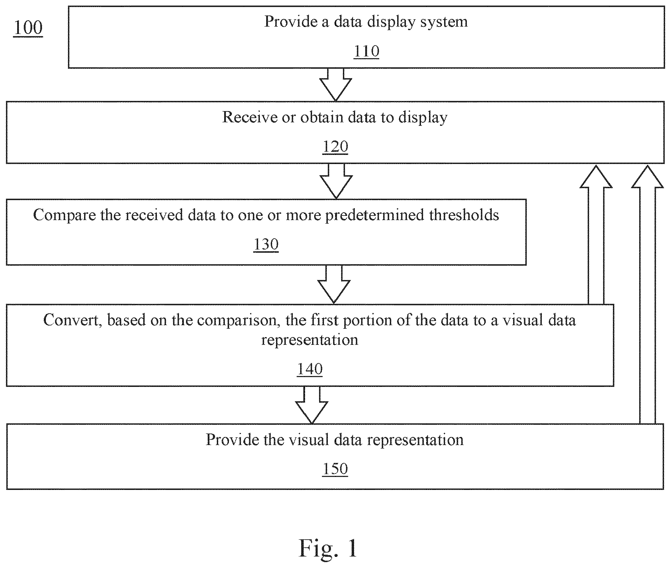

Referring to Fig. 1, in one embodiment, is a flowchart of a method 100 for displaying a visual representation of data using a data display system. The methods described in connection with the figures are provided as examples only, and shall be understood not to limit the scope of the disclosure. The data display system can be any of the systems described or otherwise envisioned herein. The data display system can be a single system or multiple different systems. According to an embodiment the data display system is a patient data display system, although other data display systems are possible.

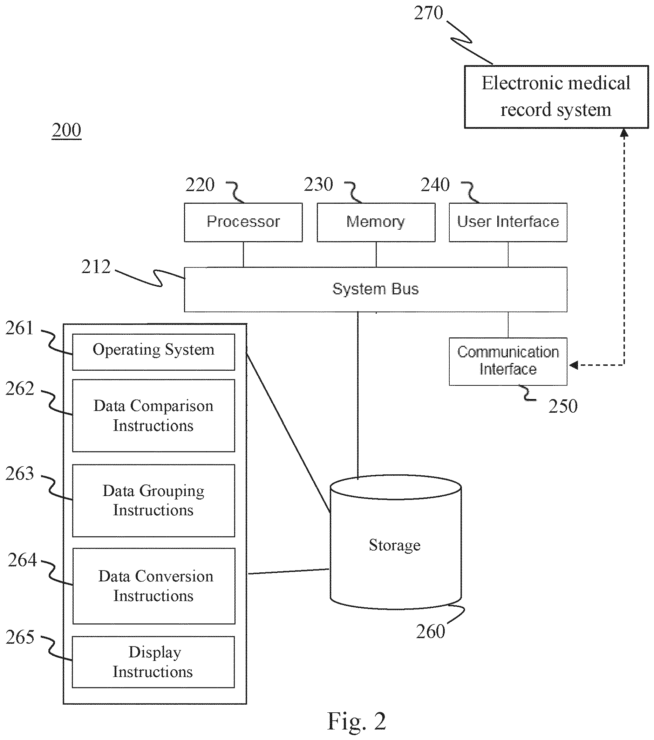

At step 110 of the method, a data display system is provided. Referring to an embodiment of a data display system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, data display system 200 can be any of the systems described or otherwise envisioned herein. Other elements and components of the data display system 200 are disclosed and/or envisioned elsewhere herein.

According to an embodiment, the data display system comprises or is in communication with a database 270 comprising the data that will be analyzed and displayed by the system. The data can be any type of data that can be analyzed and displayed as described or otherwise envisioned herein. The database 270 may be a local or remote system and can be in direct and/or indirect communication with the data display system.

According to an embodiment, the data display system is a patient data display system comprising or in communication with an Electronic Health Record (EHR) system or service 270. The EHR system or service 270 can be, for example, a database of patient data, health information, medical records, and any other information for one or more patients. Thus, the EHR database can be any database comprising patient data for one or more patients or subjects. The EHR system or service 270 may be a local or remote system and can be in direct and/or indirect communication with the patient data display system. The patient data, health information, medical records and any other information stored in the EHR database can comprise any data about a patient. For example, the patient records may comprise demographic information, diagnostic information, clinical measurements, treatment information, vital record data, and/or any other information, including over a period of time for each of the plurality of patients. Thus, the EHR system or service can be, for example, a recipient and/or source of past and current medical data for a patient, such current patient monitoring data, vital sign data, and much more.

At step 120 of the method, the data display system 200 receives, retrieves, or otherwise obtains data for one or more parameters. The one or more parameters can be any information that can be monitored or measured or otherwise obtained. According to an embodiment, the data can be received, retrieved, or otherwise obtained by the data display system 200 from any source. The source may be database 270 or any other system or source. The data can be continually or periodically fed into or requested by the data display system. The data may be temporarily stored in a local or remote database before being utilized by the data display system.

According to one embodiment, the one or more parameters are any information about a patient that can be monitored, and for which patient data can be collected or obtained. The one or more parameters for a patient can be any vital statistics, measurements, observations, or other information about a patient. As a few non-limiting examples, the one or more parameters for a patient can be body temperature, heart rate (HR), blood pressure, mean arterial pressure (MAP), central venous pressure (CVP), cardiac output, respiratory rate (RR), systemic vascular resistance (SVR), stroke volume index (SVI), SpO2, urine output, urine drain, lactate, hemoglobin, blood glucose, and a wide variety of other measurable or otherwise determined or observable patient parameters. Essentially any information about the patient and/or the patient's environment may be information that can be measured or otherwise qualified or quantified and utilized as one or more parameters for a patient.

The patient data measured or obtained for the one or more parameters may be measured or obtained by laboratory testing, by medical devices or equipment, by a clinician, or by other mechanisms. According to an embodiment, the patient data can be received, retrieved, or otherwise obtained by the patient data display system 200 from any source. The source may be the EHR system or database 270, a clinical decision support (CDS) system, or any other system or source. The patient data can be continually or periodically fed into or requested by the patient data display system. The patient data may be temporarily stored in a local or remote database before being utilized by the patient data display system.

According to an embodiment, the data is received, retrieved, or otherwise obtained by the data display system for a first time period. The first time period can be any time period sufficient to allow downstream analysis of the received data. For example, depending on the nature of the data, the time period can be very short (such as seconds or minutes or hours) or the time period can be very long (such as days or weeks or months). Data that is sampled more frequently may require a shorter time period, and data that is sampled less frequently may require a longer time period. According to one non-limiting example, a parameter can provide 1 datapoint per minute, and clinical decision making may range from 30 minutes to 8 hours, although shorter or longer is possible.

At step 130 of the method, the patient data display system compares the of received data to one or more predetermined thresholds, the one or more predetermined thresholds comprising at least a threshold separating a reference range from a first alert range. According to an embodiment, the received data comprises a plurality of time points, and every time point is compared to the predetermined threshold(s).

According to an embodiment, the predetermined thresholds comprising at least a threshold separating a reference range from a first alert range is determined by clinician, programmed by default into the data display system, received as a setting or parameter from a user interface or database, or otherwise predetermined. According to one example, one or more of the reference range, the first alert range, and/or the threshold separating the ranges may be automatically determined, and thus the range can be automatically determined, be determined based on the parameter involved (utilizing previous information or programming), or be determined based on received user input.

According to an embodiment, the reference range, which can be interpreted according to one embodiment as a normal range for a data parameter, can be surrounded on one side by a first alert rage and/or on the other side by second alert range. When the data falls outside the reference range by falling below a threshold separating the reference range from an alert range, the parameter might be interpreted according to one embodiment as being too low and may signal that an intervention or alarm state is necessary, depending on the parameter involved. When the data falls outside the reference range by exceeding a threshold separating the reference range from an alert range, the parameter might be interpreted according to one embodiment as being too high and may signal that an intervention is necessary, depending on the parameter involved.

According to another embodiment, the one or more thresholds further comprises an upper intermediate threshold separating a first alert range located above the reference range from an upper alert range located above the first alert range. The one or more thresholds can further comprise a lower intermediate threshold separating a second alert range located below the reference range from a lower alert range located below the first second range. Thus, when the data falls within the upper alert range, the parameter might be interpreted according to one embodiment as becoming or approaching too high a value, and may signal that an intervention or other action is or might be necessary at a future point, depending on the parameter involved. Similarly, when the patient data falls within the lower alert range, the parameter might be interpreted according to one embodiment as becoming or approaching too low a value, and may signal that an intervention or other action is or might be necessary at a future point, depending on the parameter involved.

According to an embodiment, patient monitors warn a caregiver when a physiological parameter of the patient is in a range that should raise a concern. As just one example, the intensive care unit (ICU) department of a hospital will have default alarm settings for each patient parameter indicating when alarms should occur. Many patient parameters can give "yellow alarms" and "red alarms," which often result in blinking yellow and blinking red, respectively, on a patient monitor. According to some embodiments, they can be accompanied by a sound as well. Typically, a red alarm is more concerning for the patient's condition than a yellow alarm. In the default alarm settings of a hospital, in accordance with one embodiment, there is typically no alarm when the patient parameters are within their normal ranges, there is a yellow alarm when the parameter is just outside that normal (or reference") range (either below or above the normal range), and there is a red alarm when the parameter is far outside the normal (or reference") range.

As an example, for heart rate, the default alarm settings could be the following:
no alarm when the heart rate is between 60 and 90 bpm (central range);
yellow alarm when the heart rate is between 50 and 60 bpm (lower intermediate range) or between 90 and 120 bpm (upper intermediate range); and
red alarm when the heart rate is below 50 (lower threshold) or above 120 bpm (upper threshold).

Likewise, for mean arterial pressure (MAP), the defaults could be the following:
no alarm for a MAP between 70 and 100 mmHg (central range);
yellow alarm when the MAP is between 60 and 70 mmHg (lower intermediate range) or between 100 and 110 mmHg (upper intermediate range); and
red alarm when the MAP is below 60 (lower threshold) or above 110 mmHg (upper threshold).

According to an embodiment, a caregiver can change the alarm settings for a particular patient. For example, what is a normal range for the population at large might not be the normal range for a specific individual, or because the parameter is influenced by the specific treatment of the patient. The ranges and thresholds can be modified by a caregiver via a user interface of the system, or of another system that communicates the modifications to the patient data display system. According to an embodiment, the ranges and thresholds are determined on an institutional level. Many other options for determining and adjusting ranges and thresholds are possible.

According to an embodiment, once the data is compared to the one or more predetermined thresholds for the parameter, the results of the comparison can be utilized immediately, or can be stored in local and/or remote database for subsequent downstream use by the system.

According to one embodiment, the data display system can group a first portion of the patient data in a first group, where all of the first portion of the patient data is within the expected reference range or the first alert range (or within any of the other ranges defined by the one or more predetermined thresholds), as determined by the comparisons from step 130 of the method. For example, the data display system can group a data point or multiple data points into a grouping since that data point or multiple data points were all within the reference range, the first alert range, or some other range after comparison to the one or more predetermined thresholds. According to one non-limiting example, a parameter can provide one datapoint per minute, such as for continuously monitored parameters like heart rate and SpO2. After determining to which range a datapoint belongs, it can be displayed on the accompanying line (i.e. elevation) and the width can be from half a minute before till half a minute after the time of the measurement (among other possibilities). In this way, in case the neighboring datapoints are in the same range and also displayed from half a minute before till half a minute after their times of measurement, it will look like they are connected and form one group, but in fact, they were just determined separately. Of course, many other variations are possible.

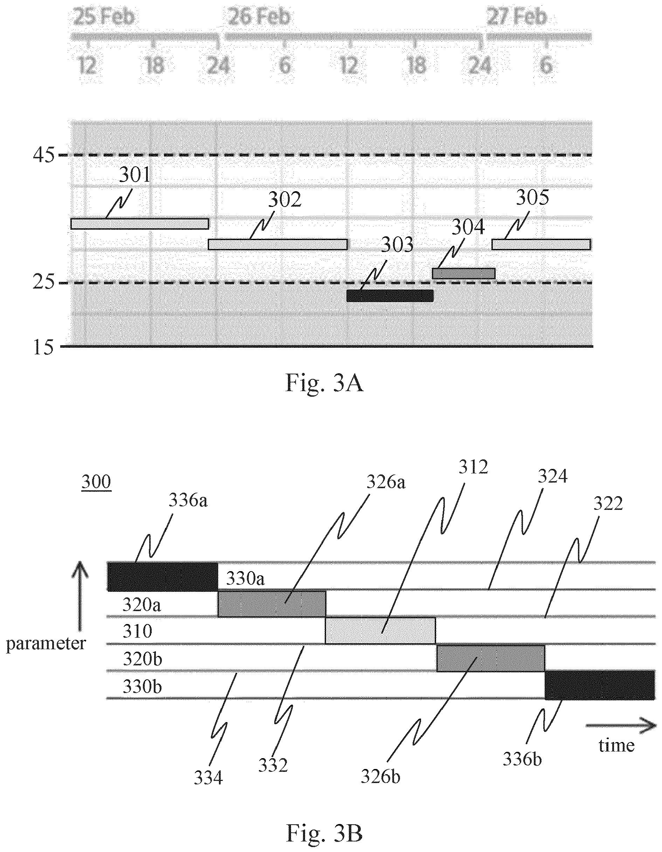

According to an embodiment, a group can fall within a single place within the reference range or an alert range. According to another embodiment, a group can fall within a plurality of different locations or places within the reference range or an alert range. For example, a group may comprise a value or values in the first alert range immediately adjacent the threshold separating the reference range from the first alert range, and can thus be located or displayed (in subsequent steps of the method) immediately adjacent this threshold. However, the first group may alternatively comprise a value or values in the first alert range further from (e.g., further above or greater than, or further below and less than) the threshold separating the reference range from the first alert range, and can thus be located or displayed (in subsequent steps of the method) further away from this threshold. Accordingly, the greater the difference of the value(s) of the first portion of the data relative to the reference range, the further away from the reference range the first portion of the data is placed within the first alert range (or other ranges), such as in a visual data representation. Referring to Fig. 3A, in one embodiment, is a schematic representation showing how a graph might be utilized for a visual data representation. In this representation, the graph comprises data in the reference range (301, 302, and 305), and contains data in a first alert range 304 and a second alert range 305. Any of these ranges can be represented simply by the location on the graph, by different colors, by different textures, and/or by a variety of other different display mechanisms.

According to an embodiment, the grouping of patient data into a group can be done multiple times for different portions of the data, such as progressive windows or other predetermined or experimentally derived subunits or portions of the data. For example, a first portion of the received data can be grouped into a first group because the first portion of the patient data is within the same range. Then a successive portion of the received data is grouped into a second group because the successive portion of the patient data is within the same range (which may be the same or different from the range of the first portion). And so on.

There are also parameters that are intermittently measured (such as hemoglobin concentration, lactate concentration, and extravascular lung water). According to an embodiment, one preferable way to visualize these data is as single blocks, not connected to an earlier and/or later measurement. Those blocks could for example have the width of one or a few minutes, while the measurements are typically separated one or more hours in time.

According to an embodiment, the patient data can be utilized immediately, or can be stored in local and/or remote database for subsequent downstream use by the system.

At step 140 of the method, the data display system converts data to a visual data representation. According to an embodiment, the first visual data representation comprises at least one of: (i) a reference range location on a visual graph when the first portion of the data is within the reference range; or (ii) a first alert range location on the visual graph when the first portion of the data is within the first alert range, where the first alert range location on the visual graph is above or below the reference range location on the visual graph.

According to an embodiment, the received data for a parameter is converted, based on the comparison to the one or more predetermined thresholds, to one of a variety of different visual data representations.

As a first example, data received by the data display system may fall within the reference range. Thus, the visual data representation for that data will comprise the reference location on a visual graph. According to one embodiment, the reference location is a central location on a visual graph. However, according to other embodiments, the reference location is a lower or upper location on a visual graph, depending on the parameter and possible ranges for that parameter.

According to an embodiment, data in the reference location on the visual graph can, but need not, be or comprise a first color. The first color can be any color, including but not limited black, gray, and other colors. However, the data in the reference location on the visual graph can comprise a pattern or other visual cue or indicator, such as brightness and darkness of the data, to indicate that the data is located within the reference location.

As a second example, data received by the data display system may be found to exceed a threshold separating the reference range from an alert range, where in this example the alert range is located above the reference range. Thus, the visual data representation for this data will comprise an elevated location on the visual graph.

According to an embodiment, the data in the alert range location on the visual graph can, but need not, be, or comprise a second color. The second color can be any color, but is notably different than the first color. Thus, according to an embodiment, the second color is configured to be visibly distinguishable from the first color. For example, the second color can be red or any other color other than the first color.

As a third example, data received by the data display system may be found to fall below a threshold separating the reference range from another alert range, where in this example the alert range is located below the reference range. Thus, the visual data representation for this data will comprise a lowered location on the visual graph and will have, be, or comprise the second color. According to an alternative embodiment, the visual data representation for this time point could comprise another color different from the first color and the second color.

As a fourth example, data received by the data display system may be found to fall within an upper alert range as described above. Thus, the visual data representation for this data will comprise an upper alert range location which is an elevated location on the visual graph above the first alert range location.

According to an embodiment, the upper alert range location on the visual graph will have, be, or comprise a third color. The third color can be any color, but is notably different than the first color or the second color. For example, the third color can be yellow or any other color other than the first color and the second color.

As a fifth example, data received by the data display system may be found to fall within the lower alert range as described above. Thus, the visual data representation for this data will comprise a lower alert range location which is a lower location on the visual graph below a first alert range below and abutting the reference range.

According to an embodiment, the lower alert range location on the visual graph will have, be, or comprise the third color. According to an alternative embodiment, the visual data representation for this time point could comprise another color different from the first color, the second color, and the second color.

According to an embodiment, rather than a first color, second color, and third color, the visual data representations may comprise other mechanisms for displaying the same information. For example, the visual data representations may comprise different patterns, variations in brightness and/or darkness, or other different designs that provide the same information as different colors.

According to an embodiment, once a data grouping is converted to a visual data representation, the results of the conversion can be utilized immediately, or can be stored in local and/or remote database for subsequent downstream use by the system.

At step 150 of the method, the data analysis system displays the visual data representation on a display. The display may be a component of the data analysis system, or may be a display in communication with the data analysis system. According to an embodiment, the display may be a display or screen of a smartphone, wearable device, laptop, portable computer, desktop, or any other device or system. According to one embodiment, the display may be a component of a patient monitor in a healthcare setting. The patient monitor may be local to or remote from the patient it is monitoring. Many other types of displays and systems are possible.

According to an embodiment, the display provided by the system comprises a visual data representation for a parameter for a first time period. The first time period may be any time period, such as a period of time leading up to the present moment. Thus, the display may provide a running display of the parameter as it is obtained, compared, converted, and displayed in near real-time. Alternatively, the display may provide a selected or predetermined time period in which to provide the patient data for the parameter, such as one hour, one day, or any other time period.

According to an embodiment, the display provided by the system comprises a visual data representation for each of a plurality of different parameters. For example, the display may comprise the visual data representation for heart rate, respiratory rate, MAP, and SpO2 all on a single display or screen. According to an embodiment, each of these individual visual data representations may comprise an individual section of the single display or screen.

According to an embodiment, the display further comprises an interactive option to change the display to provide an exact value of one or more of the visual data representations for a parameter for each of the plurality of time points for which patient data was received. For example, the heart rate visual data representation may comprise a clickable or otherwise interactive option or element that allows the visual data representation to modify in order to show the values of the patient heart rate data for some or all of the plurality of time points, where these values are shown in a traditional state-of-the-art graph with a vertical axis. This may be in addition to the visual data representation, or may be in place of the visual data representation. The clickable or otherwise interactive option or element can be a caret, or the identification of the patient parameter itself may be clickable or selectable or otherwise interactive. For example, the label "HR" or "Heart Rate" may be clickable or selectable or otherwise interactive, or may have a caret or other clickable or selectable element associated with it. As another example, the visual data representation of the patient parameter may be clickable or selectable or otherwise interactive at points or all along its length. As another example to show values, there can be a slider that can be placed at or moved by the user to a certain time and then the value of the parameter(s) at that time can be shown as a number. Many other options for identifying a visual data representation for expansion or conversion to individual data points are possible.

Referring to Fig. 1, the method may return to one or more previous steps from any other step of the method, and can comprise multiple iterations of one or more steps. For example, after converting data to a visual data representation in step 140, the system can return to step 120 or step 130 to process more data. As another example, after providing a visual data representation, the system can return to step 120 or step 130 to process more data, and can then update the visual data representation. Other variations are possible.

Referring to Fig. 3B, in one embodiment, is a schematic representation showing how a graph 300 might be utilized for a visual data representation. This embodiment comprises a 5-line collapsed view for the parameter. Over time, values for a parameter can change from a value far above normal range to a value far below normal range. Notably, a "collapsed" or "compact" view, terms which are interchangeable, refers to a visualization of data according to the embodiments described or otherwise envisioned herein, such as that shown in Fig. 3B. In contrast, an "expanded" or otherwise non-collapsed or non-compact view refers to a full graph of the data.

The graph 300 in Fig. 3B comprises a normal or reference range 310, an upper first alert range 320a, an upper alert range 330a, a lower first alert range 320b, and a lower alert range 330b. The normal or reference range is bordered by the lower boundary 322 of the upper first alert range 320a and the upper boundary 332 of the lower first alert range 320b. The upper first alert range 320a is bordered by the lower boundary 322 and the upper threshold 324. The lower first alert range 320b is bordered by the upper boundary 332 and the lower threshold 334. The upper alert range 330a is bordered by the upper threshold 324, and the lower alert range 330b is bordered by the lower threshold 334.

Notably, although the visualization depicted in Fig. 3B comprises a plurality of boundary lines (334, 332, 324, 322), which may or may not be associated with a threshold, these lines are not necessary to the data visualization. Indeed, the data displayed in Fig. 3B would be identical with or without the boundary lines (334, 332, 324, 322). Further, the word "line" may refer to the vertical or elevation location to which the data is plotted, representing a range. Thus, the lines are represented by the vertical locations 330a, 320a, 310, 320b, and 330b. Each data point is projected onto one of these elevation locations. As this happens for different time points, which correspond to different horizontal positions, this leads to five lines on which the data are projected, in this particular embodiment.

In accordance with an embodiment, when the data (i.e., the visual data representation of the data) is in the upper alert range 330a, this means that the value is far above the normal or reference range (and when connected to the alarm settings, this could correspond to a red alarm). In an embodiment, this may be associated with a red color, although other colors, patterns, brightness/darkness, and visual clues or indicators are possible. In accordance with an embodiment, when the patient data is in the lower alert range 330b, this means that the value is far below the normal or reference range (and when connected to the alarm settings, this could correspond to a red alarm). In an embodiment, this may be associated with a red color, although other colors, patterns, brightness/darkness, and visual clues or indicators are possible. In accordance with an embodiment, when the patient data is in the upper first alert range 320a, this means that the value is just outside the normal or reference range (and when connected to alarms, this could correspond to a yellow alarm). In an embodiment, this may be associated with a yellow color, although other colors, patterns, brightness/darkness, and visual clues or indicators are possible. In accordance with an embodiment, when the patient data is in the lower first alert range 320b, this means that the value is just outside the normal or reference range (and when connected to alarms, this could correspond to a yellow alarm). In an embodiment, this may be associated with a yellow color, although other colors, patterns, brightness/darkness, and visual clues or indicators are possible. In accordance with an embodiment, when the patient data is in the normal or reference range 310, this means that the patient data is in the normal or reference range. In an embodiment, this may be associated with a gray color, although other colors, patterns, brightness/darkness, and visual clues or indicators are possible.

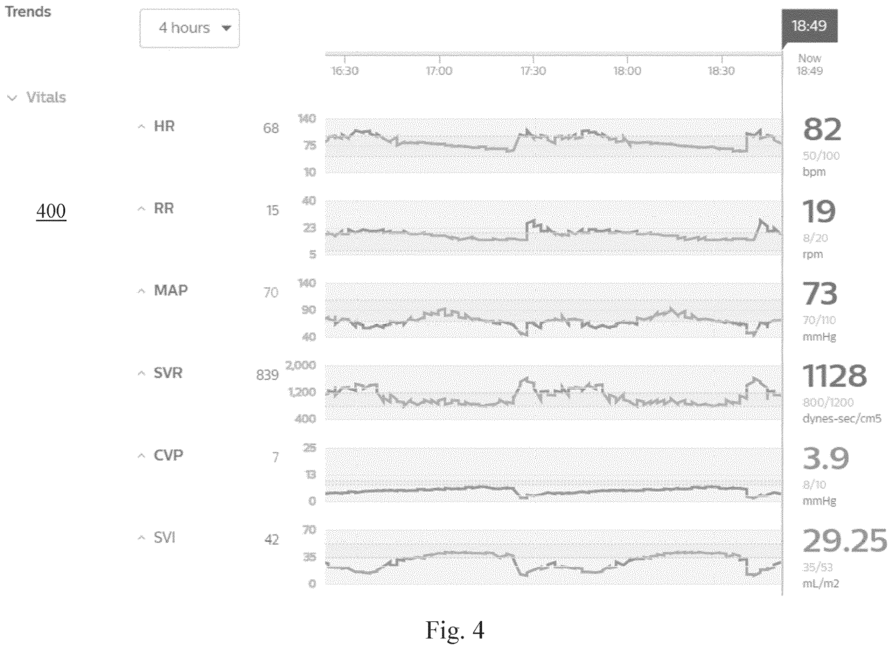

Referring to Fig. 4, in one embodiment, is an expanded view 400 of a display comprising patient data for a variety of different patient parameters, including heart rate (HR), respiratory rate (RR), mean arterial pressure (MAP), systemic vascular resistance (SVR), central venous pressure (CVP), and stroke volume index (SVI). This is not a compact or efficient view, as every patient parameter shows the value of every data point at each time point.

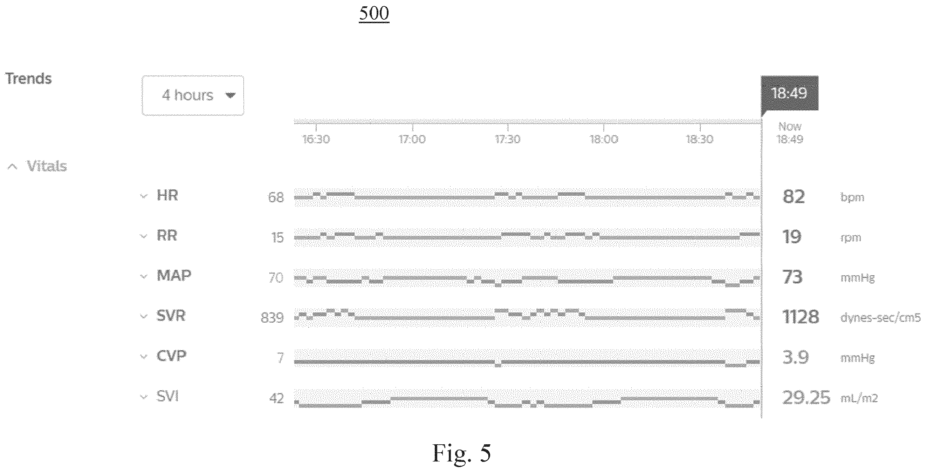

The same data is shown in the graph 400 in Fig. 5, in which the data is processed by the patient data display system according to the methods described or otherwise envisioned herein. The display comprises the same patient data for the same patient parameters, including heart rate (HR), respiratory rate (RR), mean arterial pressure (MAP), systemic vascular resistance (SVR), central venous pressure (CVP), and stroke volume index (SVI). In Fig. 5, however, the data is converted to the visual data representation, including both color (not shown but as described herein) and location on the graph (central region, intermediate lower range, intermediate upper range, upper range, or lower range). Whether a value is inside or outside the central range is indicated by color, and whether the value is above or below the central range is indicated by its location above or below the central region of the graph. Notably, a comparison of Figs. 4 and 5 demonstrate that Fig. 5 is far more compact and significantly easier and more efficient to visualize, conceptualize, interpret, and understand.

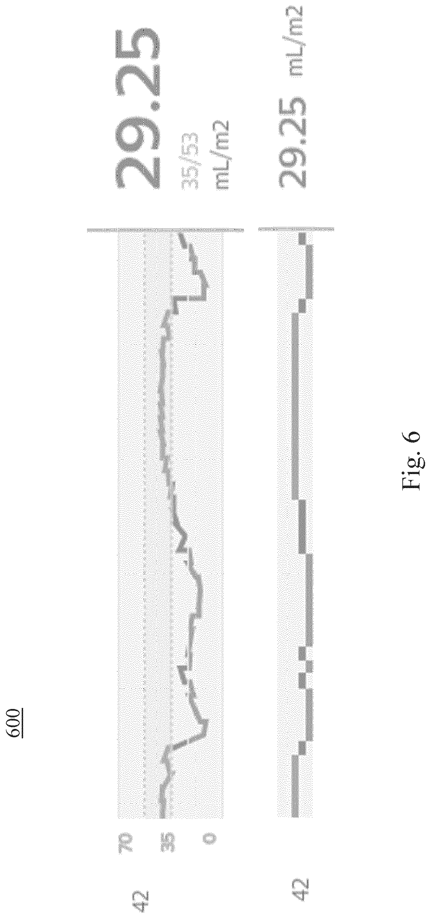

Referring to Fig. 6, in one embodiment, is a close-up view 600 of the data from Figs. 4 and 5 for a portion of the stroke volume index (SVI) data. In this close-up view, the visual data representation (from Fig. 5) in the lower panel is the compact view of the patient data (from Fig. 4) of the upper panel. The highs and lows and normal values of the patient data from the upper panel is represented in the color (not shown but as described herein) and location - upper, central, or lower region - of the visual data representation in the lower panel.

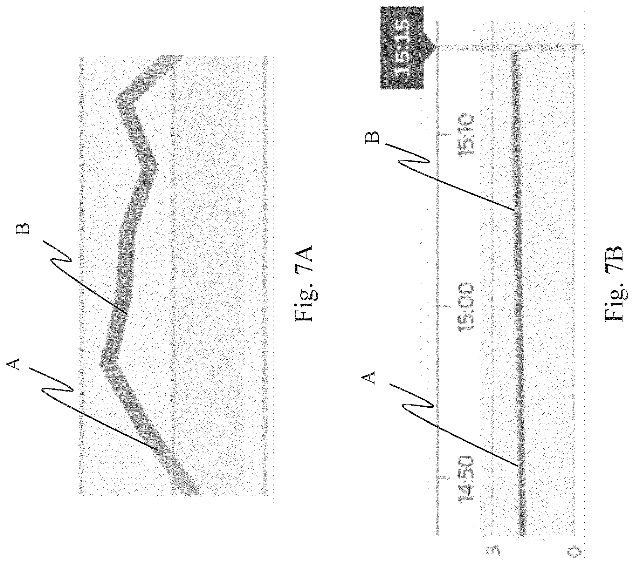

Referring to Figs. 7A and 7B are representations of patient data (Fig. 7A) such as from Fig. 4 and visual data representations (Fig. 7B) such as from Fig. 5. In Fig. 7A, the transitions between colors A and B are vertical, and in Fig. 7B the transitions between colors A and B are horizontal. According to an embodiment, a vertical transition may be preferred over a horizontal transition because when switching between expanded and collapsed view this gives the impression of looking at the same data, while the horizontal transition might be perceived as representing other data. However, other options are possible.

According to an embodiment, in the 5-line format, all five lines may have equal thickness. However, in accordance with an embodiment, the lines corresponding to the red alarms (i.e. the top and bottom line) could be thicker and the line corresponding to the normal range (i.e. the middle line) could be thinner. Notably, this difference in thickness of lines is not necessarily related to how large a range is, but can instead provide emphasis to a certain range, among other options. Other embodiments are possible.

According to an embodiment, one or more parameters may be displayed with thicker lines than one or more other parameters, in order to give that parameter more emphasis (e.g. such as because a parameter with a thicker line is related to deterioration of the patient, etc.). Which parameter(s) are displayed with thicker lines may change over time, because at one point in time, other parameters may be important than at another point in time.

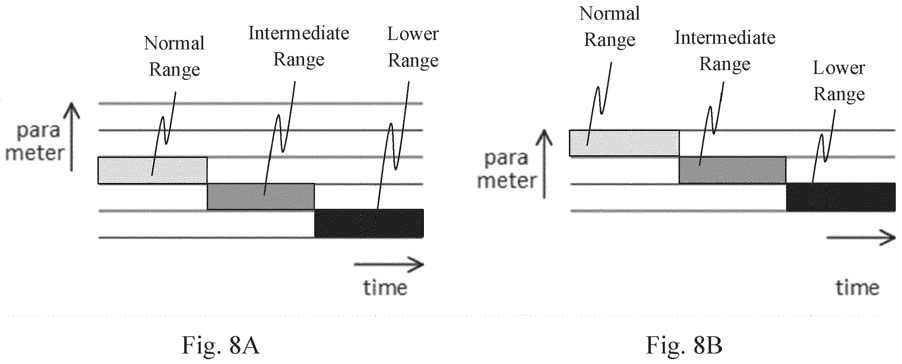

According to an embodiment, some patient parameters may only generate alarms when the patient data for that patient parameter falls below the normal range and other patient parameters may only generate alarms when they get above the normal range. As just one example, for adults, a default hospital setting could be that there would be a red alarm when SpO2 gets below 90%, a yellow alarm for a SpO2 value between 90% and 95%, and no alarm when SpO2 is above 95% (even up to 100%). There are thus only three, rather than five, ranges of importance. Although those three levels could just be depicted in a 5-lines collapsed view (not using the upper two lines) as shown in Fig. 8A, according to another embodiment the size could be reduced even more, and the graph would encompass only three lines on which the ranges are depicted as in Fig. 8B. As with the example of SpO2, grey would be used for the top line, yellow for the middle line, and red for the bottom line, although many other options are possible.

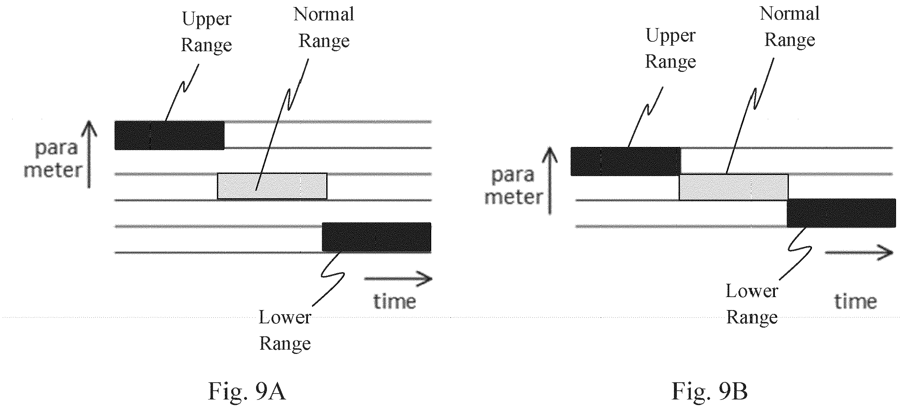

In accordance with another embodiment, the visual display may not comprise intermediate ranges (e.g., yellow alarms), and thus it might go from upper and lower ranges (e.g., red alarms) to the central or normal range (e.g., gray). Such a system might use the 5-line (Fig. 9A) or the 3-line format (Fig. 9B).

According to an embodiment, utilizing a 5-line collapsed view for patient parameters might be utilized in order to keep the view consistent with the other parameters, especially if the screen is already large enough to show all parameters of interest. On the other hand, the 3-line collapsed view might be chosen (even in between parameters on 5-line collapsed view) for clarity such as to show that there are no other levels and/or to fit more parameters on the screen.

The ranges do not need to correspond to alarm settings. According to an embodiment, the thresholds that distinguish the ranges could be set separately. In one example, these ranges reflect goals that are being set independent of the alarm settings. There may also be parameters that have no alarms at all. Still thresholds to distinguish ranges could be set. The colors may also differ from red, yellow and grey, and the number of lines on which the ranges are depicted might differ from 1, 3, or 5. For example, a rainbow scheme could be used for a 6-line representation, where the highest line shows in red heart rates above 190, the second line shows in orange heart rates between 175 and 190, the third line in yellow 145<HR<175, the fourth line in green 100<HR<145, the fifth line in blue 60<HR<100, and the bottom line in purple heart rates below 60. Such a representation could be used in a sports setting, with no alarms involved, but instead to show the athlete or his coach after the training multiple parameters of the training like heart rate, speed, cadence, etc.

As described elsewhere herein, in addition to utilizing different colors, the systems and methods described or otherwise envisioned herein could utilize different patterns (e.g., full lines vs dashed lines vs dashed-dotted-lines), variations in brightness/darkness, and other visual mechanisms. Or, when thicker lines are used, there could be differences by using a full fill, a diagonal lining, crossed lining, horizontal lining, vertical lining, and so on inside the thick line. These alternatives could be used instead of or in addition to different colors.

According to an embodiment, the colors or other differentiators could also be derived from the reference/normal range of a biomarker (e.g., Hb, Male, 8,7 - 11,2 mmol/l (equal to 140g/l - 190g/l) or from a guideline/protocol (e.g. when to provide red-blood-cells, <70g/l when not bleeding or <100g/l when bleeding is not controlled). The combination of the above two ranges could be combined in a `more than 5 line' collapsed view, for example.

According to an embodiment, the color/line pattern/filling pattern setting and/or elevation could also be based on the quality of the measurement. This could be related to technical alarms (so called `blue alarms', such as `leads off'; for which for example a blue color could be used), to signal to noise ratio, or to expected accuracy in general. For example, when bad perfusion is measured with an SpO2 sensor, the SpO2 value is also expected to be unreliable, and this can be reflected in the color/pattern of the line/filling.

The physiological parameters and interventions can be depicted along a horizontal time axis; the time axes of the parameters and interventions correspond to each other. However, according to another embodiment, the horizontal axis could instead be one of step in protocol, kind of intervention, ventilator setting, and other options. It is also possible that the time axis is vertical instead of horizontal. The latest parameter range might then be shown at the top of the vertical bar and the vertical bars that show the complete timeline of the different parameters are shown next to each other.

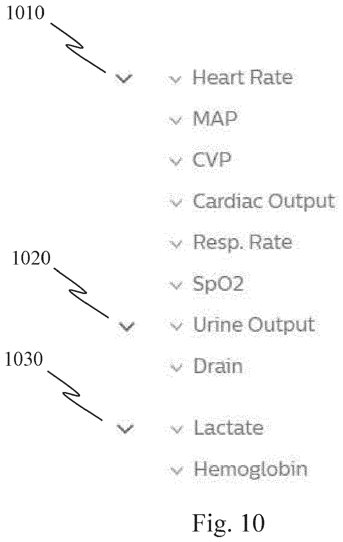

In accordance with an embodiment, in addition to the optional ability to expand and collapse each individual patient parameter using an interactive element, groups of parameters could be made that can change from collapsed to expanded or back all together by one click, as shown in Fig. 10. With the (three) leftmost carets in Fig. 10, groups of parameters can be expanded all at once. When pressing the top left caret 1010, all continuously measured parameters (HR, MAP, CVP, CO, RR, SpO2) will expand to their full graphs. When pressing the middle left caret 1020, the fluid out parameters (urine output and drain) will expand to their full graphs. When pressing the lower left caret 1030, the lab values (lactate and hemoglobin) will expand to their full graphs.

In accordance with an embodiment, the system can comprise the option to choose for a certain patient profile, including either manually by a caregiver or retrieved from an EMR, among other possibilities. In this embodiment, defaults are set per patient profile for which parameters are expanded and which are collapsed in the initial view. For example, for a patient with COPD, parameters respiration rate and SpO2 can be automatically expanded, while those parameters are collapsed for a patient without lung disease. There could also be a default setting where parameters that normally don't vary significantly (such as SpO2 and CVP) are automatically collapsed, while parameters that may vary considerably, such as MAP and HR, are automatically expanded. These could just be the initial settings, which can be manually adapted by a caregiver during the patient's stay.

In accordance with an embodiment, as soon as a parameter crosses a certain threshold and thereby getting further out of normal range, the view of that parameter is expanded. In this way, the caregiver can see how fast the (potentially critical) parameter is dropping/increasing. The threshold might coincide with a color-change boundary (e.g., the parameter gets expanded as soon as it enters the red range) or might have a different value. When crossing back over the threshold, the parameter might automatically collapse. The crossing back threshold (which changes expanded to collapsed) might have a different value than the crossing forth threshold (which changes collapsed to expanded).

The methods and systems described herein are primarily described in conjunction with a patient monitor in which the parameters are physiological signals such as the regular vital signs (heart rate, blood pressure, respiration rate, oxygen saturation (SpO2), and core body temperature), advanced hemodynamic parameters (cardiac output, stroke volume, systemic vascular resistance, etc.), or other physiological signals (urine output, skin temperature, etc.). However, the methods and systems described herein can similarly be utilized for applications outside healthcare, including applications unrelated to human parameters. For example, the methods and systems described herein could be utilized for parameters of a car during driving, like speed, temperature of the engine, engine revolutions per minute, among other parameters. And this is just one non-limiting example of other applications of the methods and systems described herein.

Referring again to Fig. 2, in one embodiment, is a schematic representation of a data display system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display such as a touchscreen display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, system 200 comprises or is in communication with a local or remote database 270, which is a database from which the data may be obtained. According to an embodiment, the database 270 is an electronic medical records database from which patient data is obtained. The database may be a local or remote database and is in direct and/or indirect communication with system 200. Thus, according to an embodiment, data display system 200 comprises a database or other data system 270.

According to an embodiment, storage 260 of system 200 may store one or more algorithms, modules, and/or instructions to carry out one or more functions or steps of the methods described or otherwise envisioned herein. For example, the system may comprise, among other instructions or data, data comparison instructions 262, data conversion instructions 263, and display instructions 264.

According to an embodiment, data comparison instructions 262 direct the system to compare some or all of received data to one or more predetermined thresholds. According to an embodiment, the received data comprises a plurality of time points, and every time point is compared to the one or more predetermined thresholds. According to another embodiment, only some of the time points are compared to the one or more predetermined thresholds. For example, the system may only sample the received data, or may otherwise filter or preprocess the received data such that fewer than all received data points are compared to the one or more predetermined thresholds.

According to an embodiment, the one or more predetermined thresholds are determined by clinician, programmed by default into the data display system, received as a setting or parameter from a user interface or database, or otherwise predetermined. According to one example, a threshold may be automatically determined, and thus a range can be automatically determined. Alternatively, a threshold and/or a range may be based on the parameter involved (utilizing previous information or programming), or based on received user input.

According to an embodiment, data conversion instructions 263 direct the system to convert the data to a visual data representation as described or otherwise envisioned herein. According to an embodiment, patient data is converted, based on the comparison to the one or more predetermined thresholds, to one of a variety of different visual data representations as described or otherwise envisioned herein.

According to an embodiment, display instructions 264 direct the system to display the visual data representation on a display. According to an embodiment, the display may be a display or screen of a smartphone, wearable device, laptop, portable computer, desktop, or any other device or system. According to one embodiment, the display may be a component of the patient data analysis system, or may be a display in communication with the patient data analysis system. The display may be a component of a patient monitor in a healthcare setting. The patient monitor may be local to or remote from the patient it is monitoring.

According to an embodiment, the display provided by the system comprises a visual data representation for a parameter for a first time period. The first time period may be any time period, such as a period of time leading up to the present moment. Thus, the display may provide a running display of the parameter as it is obtained, compared, converted, and displayed in near real-time. Alternatively, the display may provide a selected or predetermined time period in which to provide the data for the parameter, such as one hour, one day, or any other time period. According to an embodiment, the display provided by the system comprises a visual data representation for each of a plurality of different parameters. According to an embodiment, the display further comprises an interactive option to change the display to provide an exact value of one or more of the visual data representations for a parameter for each of the plurality of time points for which patient data was received.

According to an embodiment, the data display system is configured to process many thousands or millions of datapoints in the comparison of data to the one or more predetermined thresholds, and in the conversion of the data to the visual data representation, and in the display of that visual data representation. Generating the display requires processing of millions of datapoints from input data and the resulting analysis. This can require millions or billions of calculations to generate the comparison, the conversion, and the resulting display. Thus, generating and providing the display comprises a process with a volume of calculation and analysis that a human brain cannot accomplish in a lifetime, or multiple lifetimes.

In addition, the system is configured to continually receive new data, perform the analysis, and provide periodic or continual updates via display. This requires the analysis of thousands or millions of datapoints on a continual basis to optimize the reporting, requiring a volume of calculation and analysis that a human brain cannot accomplish in a lifetime.

Furthermore, the novel data display system as described or otherwise envisioned herein provides a significant technological improvement and advantage over prior art displays and display systems. The display described or otherwise envisioned herein is significantly more compact and efficient than prior art systems, which provides at least two improvements and advantages. First, the display can render more efficiently and more quickly as the compact view (referring to Fig. 5 for example) provides the same information as the expanded view (referring to Fig. 4 for example), but does so with less screen space, thus resulting in quicker and more efficient rendering. Second, the display can be more easily read and interpreted and understood by a user such as a clinician, which can improve patient care. Thus, the novel display and patient data display system as described or otherwise envisioned herein has an enormous positive effect on patient analysis and care compared to prior art systems.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for displaying a visual representation of data, comprising:
receiving (120) data representing a measured parameter for a plurality of time points, wherein the data is variable over time;
comparing (130) the received data representing a measured parameter to one or more predetermined thresholds at each of the plurality of time points, wherein the one or more predetermined thresholds comprises a threshold separating a reference range from a first alert range;
converting (140), based on the comparison, the received data into a first visual data representation, wherein the first visual data representation comprises at least one of: (i) a reference range location on a visual graph when the received data is within the reference range; or (ii) a first alert range location on the visual graph when the received data is within the first alert range, wherein the first alert range location on the visual graph is above or below the reference range location on the visual graph;
displaying (150) the first visual data representation on a display.

2. The method of claim 1, further comprising repetitions of the method to display a plurality of converted visual data representations on the display, each visual data representation representing a different measured parameter.

3. The method of claim 1, wherein the first alert range is an alarm range.

4. The method of claim 1, wherein the greater a difference of a value of the data relative to the reference range, the further away from the reference range the data is placed within the first alert range in the first visual data representation.

5. The method of claim 1, wherein the first visual data representation comprises a first color, pattern, brightness, darkness, or thickness when the data is within the reference range, and comprises a second color, pattern, brightness, darkness, or thickness when the data is within the first alert range, the first color, pattern, brightness, darkness, or thickness and the second color, pattern, brightness, darkness, or thickness being different colors, patterns, brightness, darkness, or thickness.

6. The method of claim 5, wherein the first color is gray, and the second color is yellow or red.

7. The method of claim 1, wherein the one or more predetermined thresholds further comprises a threshold identifying a second alert range, wherein the data is within the reference range, the first alert range, or the second alert range; and wherein the first visual data representation can further comprise: (iii) a second alert range location on the visual graph when the data is within the second alert range.

8. The method of claim 7, wherein the second alert range is located above or below the reference range location on the visual graph, on a side of the reference range opposite the first alert range.

9. The method of claim 7, wherein the first alert range is located between the reference range and the second alert range on the visual graph.

10. The method of claim 7, wherein the first alert range and/or the second alert range is an alarm range.

11. The method of claim 7, wherein the first alert range and the second alert range are unequal ranges relative to each other.

12. The method of claim 1, further displaying, on the display, an interactive option to change the display to provide exact values for the data in the first visual data representation.

13. A system (200) for displaying a visual representation of data, comprising:
data representing a measured parameter for a plurality of time points, wherein the data is variable over time;
a processor (220) configured to: receive the data; compare the received data to one or more predetermined thresholds at each of the plurality of time points, wherein the one or more predetermined thresholds comprises a threshold separating a reference range from a first alert range; convert, based on the comparison the received data into a first visual data representation, wherein the first visual data representation comprises at least one of: (i) a reference range location on a visual graph when the received data is within the reference range; or (ii) a first alert range location on the visual graph when the received data is within the first alert range, wherein the first alert range location on the visual graph is above or below the reference range location on the visual graph; and
a user interface (240) configured to display the visual data representation.

14. The system of claim 13, wherein the first visual data representation comprises a first color, pattern, brightness, darkness, or thickness when the data is within the reference range, and comprises a second color, pattern, brightness, darkness, or thickness when the data is within the first alert range, the first color, pattern, brightness, darkness, or thickness and the second color, pattern, brightness, darkness, or thickness being different colors, patterns, brightness, darkness, or thickness.

15. The system of claim 13, wherein the first alert range is an alarm range.
